# EUROPEAN PATENT SPECIFICATION

(11) **EP 2 612 149 B1**
(45) Date of publication and mention of the grant of the patent: **09.08.2017**
(21) Application number: 11798771.9
(22) Date of filing: 21.06.2011
(51) Int. Cl.: G01N 33/569

(54) **COMPOSITIONS AND METHODS FOR DIAGNOSING AND MONITORING DISEASE AND TREATMENT VIA ANTIGEN-SPECIFIC MOLECULES**
ZUSAMMENSETZUNGEN UND VERFAHREN ZUR DIAGNOSE UND ÜBERWACHUNG VON ERKRANKUNGEN UND BEHANDLUNG ANHAND VON ANTIGENSPEZIFISCHEN MOLEKÜLEN
COMPOSITIONS ET MÉTHODES POUR DIAGNOSTIQUER ET SURVEILLER UNE MALADIE ET TRAITEMENT AU MOYEN DE MOLÉCULES SPÉCIFIQUES D'UN ANTIGÈNE

(30) Priority: 21.06.2010 US 356942 P
(43) Date of publication of application: 10.07.2013
(73) Proprietor: Innatoss Laboratories B.V., 5349 AB Oss (NL)
(72) Inventor: BIEGER, Wilfried P., 80333 Munich (DE); KELLERMANN, Gottfried, H., Osceola, Wisconsin 54020 (US)
(74) Representative: De Vries & Metman
(86) International application number: PCT/US2011/041288
(87) International publication number: WO 2011/163258

(56) References cited:
- US-A- 5 853 987
- US-A1- 2004 115 744
- US-A1- 2005 244 421
- US-A1- 2009 297 560
- TOKARSKA-RODAK M ET AL: "The detection of antibodies directed against specific antigens of Borrelia burgdorferi sensu lato (B. burgdorferi s.s., B. afzelii, B. garinii, B. spielmanii) in patients with borreliosis in Eastern Poland", INTERNATIONAL JOURNAL OF INFECTIOUS DISEASES, INTERNATIONAL SOCIETY FOR INFECTIOUS DISEASES, HAMILTON, CA, vol. 14, 1 March 2010 (2010-03-01), pages e387-e388, XP026992708, ISSN: 1201-9712 [retrieved on 2010-03-01]
- ZAJKOWSKA J ET AL: "The usefulness of 'in vivo' antigens in the diagnosis of human Lyme borreliosis", INTERNATIONAL JOURNAL OF MEDICAL MICROBIOLOGY, URBAN UND FISCHER, DE, vol. 298, 1 September 2008 (2008-09-01), pages 361-364, XP023520187, ISSN: 1438-4221, DOI: 10.1016/J.IJMM.2008.03.009 [retrieved on 2008-05-22]
- PIETERNELLA MARIA HOUTMAN ET AL: "Borrelial fasciitis: as yet insufficient proof for a distinct entity", RHEUMATOLOGY INTERNATIONAL ; CLINICAL AND EXPERIMENTAL INVESTIGATIONS, SPRINGER, BERLIN, DE, vol. 29, no. 11, 9 June 2009 (2009-06-09), pages 1385-1388, XP019738756, ISSN: 1437-160X, DOI: 10.1007/S00296-009-0987-3
- A. G. BARBOUR ET AL: "A Genome-Wide Proteome Array Reveals a Limited Set of Immunogens in Natural Infections of Humans and White-Footed Mice with Borrelia burgdorferi", INFECTION AND IMMUNITY, vol. 76, no. 8, 1 August 2008 (2008-08-01) , pages 3374-3389, XP55000211, ISSN: 0019-9567, DOI: 10.1128/IAI.00048-08
- BAEHR ET AL.: 'Improving the in vitro antigen specific T cell proliferation assay: the use of antigen specific stiulation and decrease of bystander proliferation' JOURNAL OF IMMUNOLOGICAL METHODS. vol. 251, no. 1-2., 01 May 2001, pages 63 - 71, XP004233073
- COLLINS ET AL.: 'Immunoreactive epitopes on an expressed recombinant flagellar protein of Borrelia burgdorferi' INFECT. IMMUN. vol. 59, no. 2, February 1991, pages 514 - 520, XP055125268
- DATABASE UNIPROT [Online] 14 April 2009 FRASER-LIGGETT ET AL., XP003030103 Database accession no. 8EHN9
- DATABASE UNIPROT [Online] 03 March 2009 FRASER-LIGGETT ET AL., XP003030104 Database accession no. BEGN9
- DATABASE UNIPROT [Online] 10 February 2009 FRASER-LIGGETT ET AL., XP003030102 Database accession no. 7J019
- DATABASE UNIPROT [Online] 10 February 2009 FRASER-LIGGETT ET AL., XP003030101 Database accession no. 7J1V5
- VALENTINE-THON ET AL: "A novel lymphocyte transformation test (LTT-MELISA(R)) for Lyme borreliosis", DIAGNOSTIC MICROBIOLOGY AND INFECTIOUS DISEASES, ELSEVIER SCIENCE PUBLISHING CO., AMSTERDAM, NL, vol. 57, no. 1, 15 December 2006 (2006-12-15), pages 27-34, XP005805950, ISSN: 0732-8893, DOI: 10.1016/J.DIAGMICROBIO.2006.06.008
- 'DER MIKROBIOLOGE', [Online] 01 December 2005, pages 205 - 241, XP055201659 ISBN: 0943674X Retrieved from the Internet: <URL:http://www.baemi.de/fileadmin/baemi/de r_mikrobiologe_komplett/Mikrobiologe_2005-0 6_INTERNETVERSION.pdf> [retrieved on 2015-07-10]

## Description

### BACKGROUND

### 1. Technical Field

This document provides methods and materials related to compositions and methods for diagnosing and monitoring treatment of Lyme disease. For example, provided herein are compositions of substantially pure polypeptides, or antigenic fragments thereof, and methods of using such compositions for diagnosing Lyme disease and monitoring a subject's response to Lyme disease treatment.

### 2. Background Information

Lyme disease, or *Lyme borreliosis*, is the most prevalent tick-borne disease of humans in the United States. Lyme disease is transmitted by the bite of blacklegged ticks. Infection is caused by the bacterium *Borrelia burgdorferi* **(scnso stricto, but includes other species of *Borrelia*), resulting in an illness affecting various organ systems of the body. The clinical implications of Lyme disease can be seen in dermatologic, neurologic and rheumatologic manifestations. While there is significant variability in the presentation of Lyme disease, typical symptoms include fever, headache, fatigue, swollen lymph nodes, muscle and joint aches, and sometimes a characteristic circular skin rash called erythema migrans.

Sometimes Lyme disease can be cured with antibiotics alone, especially if treatment is begun early in the course of illness. A large percentage of patients with Lyme disease, however, have symptoms that last months to years even after treatment with antibiotics. The prolonged or recurrent symptoms of a "chronic" Lyme infection can include muscle and joint pains, arthritis, cognitive deficits, sleep disturbances, and/or fatigue.

Diagnosis of Lyme disease is often based upon a physician's review of clinical symptoms and the patient's exposure risk in an area where the disease is endemic. Prompt diagnosis and treatment of Lyme disease is the key to avoiding chronic Lyme disease and its deleterious effects. Early detection of Lyme disease can be difficult, in part because the characteristic rash may not be present and the flu-like symptoms, can be caused by many other factors which can confuse diagnosis.

Laboratory assays for identifying lyme disease have been also reported. For instance, Tokarska- Rodak et al. (International Journal of Infectious Diseases, International Society for Infectious Diseases, vol. 14, 2010, pages e387 - e388,) and Zajkowska et al. (International Journal of Medical Microbiology, vol. 298, 2008, pages 361 - 364) describe methods for detecting antibodies of Borrelia infection using western blot tests. Houtman et. al. (Rheumatology International, Clinical and Experimental Investigations, vol.29, no. 11, 2009, pages 1385 - 1388) describe methods for determining Borrelia infection using Eo-count (i.e. number of eosinophilic cells with respect to total white blood cells), Borrelia ELISA and Borrelia blot testing of peripheral blood, and Silver stain and PCR on tissue. Barbour et al. (Infection and Immunity, vol. 76, no. 8, 2008, pages 3374 - 3389) describe the use of a genome-wide proteome of *Borrelia burgdorferi* to identify new antigens, by applying an array to analysis of sera of humans and white-footed mice with Lyme borreliosis. Wiske (Der Mikrobiologe, 2005, pages 205-241) describes methods for the diagnostic of Lyme Borreliosis focusing on methods for the detection of pathogens, such as a cultivation and PCR methods and for the detection of antibodies, such as ELISA and Immunoblot. Valentine-Thon (2007 Diagnostic Microbiology & Infectious Diseases, vol. 51, no.1, 2006, pages 27-34) describes a lymphocyte transformation test (LTT) for Lyme Borreliosis wherein peripheral blood mononuclear cells (PBMCs) isolated from blood samples from patients with suspicion of Lyme infection are tested against 4 to 8 different recombinant Borrelia antigens including OspC, p.41-1, p.41-2, andp.100.
However, many available laboratory assays yield unreliable results. For example, nonspecific detection of antibodies to cross-reactive *Borrelia* antigens has contributed to false-positive laboratory results and overdiagnosis of Lyme disease. In contrast, false- negative results for subjects with weak or absent immune responses have been reported, due in part to tests performed too early in the course of the immune response. See, for review, Aguero- Rosenfeld et al., Clinical Microbiology Reviews 18(3):484-509 (2005). Overall Lyme disease is generally underdiagnosed and under-reported.

### SUMMARY

This document provides methods and materials related to antigen specific cytokine testing for diagnosing Lyme disease. This document also provides methods and materials for monitoring the response of a subject to treatment for the same.

For example, this document provides compositions for use in lymphocyte transformation and cytokine assays to detect a subject's immunestimulatory response to Lyme. As described herein, the methods and materials provided can be used for detection of previous and/or acute *Borrelia* infection.

Diagnosing Lyme disease and monitoring a subject's response to treatment for such disease according to the methods provided herein can allow a clinician or other health or research professional to better identify subjects having such responses and to optimize treatment. Such diagnostic and evaluative methods can have substantial value for clinical use.

In one aspect, this document features a composition. The composition comprises (a) a substantially purified polypeptide comprising an amino acid sequence having at least 80% sequence identity to any one of SEQ ID NOs. 1-4, or to an antigenic fragment thereof; (b) a substantially purified polypeptide comprising an amino acid sequence having at least 80% sequence identity to SEQ ID NO:5, or to an antigenic fragment thereof; (c) a substantially purified polypeptide comprising an amino acid sequence having at least 80% sequence identity to any one of SEQ ID NOs. 6-7, or to an antigenic fragment thereof; and (d) a substantially purified polypeptide comprising an amino acid sequence having at least 80% sequence identity to any one of SEQ ID NOs. 8-9, or to an antigenic fragment thereof. The composition can comprise (a) a substantially purified polypeptide comprising an amino acid sequence having at least 80% sequence identity to any one of SEQ ID NOs. 1-4; (b) a substantially purified polypeptide comprising an amino acid sequence having at least 80% sequence identity to SEQ ID NO:5; (c) a substantially purified polypeptide comprising an amino acid sequence having at least 80% sequence identity to any one of SEQ ID NOs. 6-7; and (d) a substantially purified polypeptide comprising an amino acid sequence having at least 80% sequence identity to any one of SEQ ID NOs. 8-9. The composition can comprise (a) a substantially purified polypeptide comprising SEQ ID NO:1; (b) a substantially purified polypeptide comprising SEQ ID NO:5; (c) a substantially purified polypeptide comprising SEQ ID NO:7; and (d) a substantially purified polypeptide comprising SEQ ID NO:8. The composition can comprise (a) any one of SEQ ID NOs: 1-4 in substantially purified form; (b) SEQ ID NO:5 in substantially purified form; (c) any one of SEQ ID
NOs:6-7 in substantially purified form; and (d) any one of SEQ ID NOs:8-9 in substantially purified form. The composition as provided above can further comprise any one or both of: (a) a substantially purified polypeptide comprising an amino acid sequence having at least 80% sequence identity to any one of SEQ ID NOs: 10-12, or to an antigenic fragment thereof; and (b) a substantially purified polypeptide comprising an amino acid sequence having at least 80% sequence identity to any one of SEQ ID NOs: 13-18, or to an antigenic fragment thereof The composition can further comprise Interferon-alpha (IFN-α). The composition can further comprise one or more polypeptides derived from a species selected from the group consisting of *Babesia bovis, Babesia divergens, Babesia microti, Bartonella bacilliformis, Bartonella henselae, Bartonella quintana, Bartonella rochadimae, Anaplasma phagocytophilum, Ehrlichia ewingii, Ehrlichia chaffeenis, Ehrlichia canis, Neorickettsia sennetsu, Mycoplasma fermentans, Mycoplasma hominis, Mycoplasma pneumoniae, Mycoplasma genitalium, Mycoplasma penetrans, Rickettsia rickettsii*, and *Rickettsia typhi.*

In another aspect, this document features a composition. The composition consists essentially of (a) a substantially purified polypeptide comprising SEQ ID NO:1; (b) a substantially purified polypeptide comprising SEQ ID NO:5; (c) a substantially purified polypeptide comprising SEQ ID NO:7; and (d) a substantially purified polypeptide comprising SEQ ID NO:8.

In yet another aspect, this document features a composition. The composition consists essentially of (a) a substantially purified polypeptide comprising SEQ ID NO:1; (b) a substantially purified polypeptide comprising SEQ ID NO:5; (c) a substantially purified polypeptide comprising SEQ ID NO:7; (d) a substantially purified polypeptide comprising SEQ ID NO:8; (e) a substantially purified polypeptide comprising SEQ ID NO: 10; and (f) a substantially purified polypeptide comprising SEQ ID NO: 13.

In another aspect, this document features a method for diagnosing Lyme disease in a subject. The method comprises determining a stimulation index (SI) of lymphocytes obtained from the subject. The determining can comprise contacting a composition to the lymphocytes. The composition can comprise (i) a substantially purified polypeptide comprising an amino acid sequence having at least 80% sequence identity to any one of SEQ ID NOs. 1-4, or to an antigenic fragment thereof; (ii) a substantially purified
polypeptide comprising an amino acid sequence having at least 80% sequence identity to SEQ ID NO:5, or to an antigenic fragment thereof; (iii) a substantially purified polypeptide comprising an amino acid sequence having at least 80% sequence identity to any one of SEQ ID NOs. 6-7, or to an antigenic fragment thereof; and (iv) a substantially purified polypeptide comprising an amino acid sequence having at least 80% sequence identity to any one of SEQ ID NOs. 8-9, or to an antigenic fragment thereof. The method also comprises measuring one or more cytokines in a sample from the subject. An increase in one or both of SI and cytokine level relative to a control can be indicative of Lyme disease.

The composition can further comprise any one or both of: (a) a substantially purified polypeptide comprising an amino acid sequence having at least 80% sequence identity to any one of SEQ ID NOs: 10-12, or to an antigenic fragment thereof; and (b) a substantially purified polypeptide comprising an amino acid sequence having at least 80% sequence identity to any one of SEQ ID NOs: 13-18, or to an antigenic fragment thereof. The one or more cytokines can be selected from the group consisting of IL-1 beta, IL-4, IL-6, IL-8, IL-10, IL-13, MCP-1, G-CSF, IFN-gamma, and TNF- alpha. Determination of a stimulation index can comprise measuring uptake of tritiated thymidine by the lymphocytes. Determination of a stimulation index can comprise contacting the lymphocytes to IFN-α. The measuring one or more cytokines can comprise performing a bioassay, an immunoassay, flow cytometry, carboxyfluorescein succinimidyl ester (CFSE) staining or radioimmunoassay (RIA). The immunoassay can be an enzyme-linked immunosorbent assay. The composition can further comprise one or more polypeptides derived from a species selected from the group consisting of *Borellia afzenii, Borellia garinii, Borellia miyamotoi, Babesia bovis, Babesia divergens, Babesia microti, Bartonella bacilliformis, Bartonella henselae, Bartonella quintana, Bartonella rochalimae, Anaplasma phagocytophilum, Ehrlichia ewingii, Ehrlichia chaffeenis, Ehrlichia canis, Neorickettsia sennetsu, Mycoplasma fermentans, Mycoplasma hominis, Mycoplasma pneumoniae, Mycopdasma genitalium, Mycopdasma penetrans, Rickettsia rickettsii*, and *Rickettsia typhi*.

In another aspect, this document features a method for determining the likelihood of developing symptoms associated with Lyme disease in a subject.

The method comprises determining a stimulation index (SI) of lymphocytes obtained from the subject. The determining can comprise contacting a composition to the lymphocytes. The composition can comprise (i) a substantially purified polypeptide comprising an amino acid sequence having at least 80% sequence identity to any one of SEQ ID NOs. 1-4, or to an antigenic fragment thereof; (ii) a substantially purified polypeptide comprising an amino acid sequence having at least 80% sequence identity to SEQ ID NO:5, or to an antigenic fragment thereof; (iii) a substantially purified polypeptide comprising an amino acid sequence having at least 80% sequence identity to any one of SEQ ID NOs. 6-7, or to an antigenic fragment thereof; and (iv) a substantially purified polypeptide comprising an amino acid sequence having at least 80% sequence identity to any one of SEQ ID NOs. 8-9, or to an antigenic fragment thereof. The method also comprises measuring one or more cytokines in a sample from the subject. An increase in one or both of SI and cytokine level relative to a control can be indicative of an increased likelihood of developing symptoms associated with Lyme disease. The composition can further comprise any one or both of (a) a substantially purified polypeptide comprising an amino acid sequence having at least 80% sequence identity to any one of SEQ ID NOs: 10-12, or to an antigenic fragment thereof; and (b) a substantially purified polypeptide comprising an amino acid sequence having at least 80% sequence identity to any one of SEQ ID NOs: 13-18, or to an antigenic fragment thereof. The one or more cytokines can be selected from the group consisting of IL-1 beta, IL-4, IL-6, IL-8, IL-10, IL-13, MCP-1, G-CSF, IFN-gamma, and TNF-alpha. The determining a stimulation index can comprise measuring uptake of tritiated thymidine, for example, by the lymphocytes or uptake of fluroescent antibodies which can measure lymphocyte proliferation, migration and positioning. The determining a stimulation index can comprise contacting the lymphocytes to IFN-α. The measuring one or more cytokines can comprise performing a bioassay, an immunoassay, flow cytometry, carboxyfluorescein succinimidyl (CFSE) ester staining, CD69 staining or radioimmunoassay (RIA). The immunoassay can be an enzyme-linked immunosorbent assay. The composition can further comprise one or more polypeptides derived from a species selected from the group consisting of *Borellia affzini, Borellia garinii, Borellia miyamotoi, Babesia bovis, Babesia divergens, Babesia microti, Bartonella bacilliformis, Bartonella henselae, Bartonella quintana, Bartonella rochalimae, Anaplasma phagocytophilum, Ehrlichia ewingii, Ehrlichia chaffeenis, Ehrlichia canis, Neorickettsia sennetsu, Mycoplasma fermentans, Mycoplasma hominis, Mycoplasma pneumoniae, Mycopdasma genitalium, Mycopdasma penetrans, Rickettsia rickettsii*, and *Rickettsia typhi*.

In yet another aspect, this document features a method of monitoring treatment of Lyme disease in a subject. The method comprises determining a baseline stimulation index (SI) of lymphocytes obtained from the subject. The determining can comprise contacting a composition to the lymphocytes. The composition can comprise (i) a substantially purified polypeptide comprising an amino acid sequence having at least 80% sequence identity to any one of SEQ ID NOs: 1-4, or to an antigenic fragment thereof; (ii) a substantially purified polypeptide comprising an amino acid sequence having at least 80% sequence identity to SEQ ID NO:5, or to an antigenic fragment thereof; (iii) a substantially purified polypeptide comprising an amino acid sequence having at least 80% sequence identity to any one of SEQ ID NOs:6-7, or to an antigenic fragment thereof; and (iv) a substantially purified polypeptide comprising an amino acid sequence having at least 80% sequence identity to any one of SEQ ID NOs:8-9, or to an antigenic fragment thereof. The method can also comprise measuring one or more cytokines in a sample from the subject; and comparing a later SI and cytokine level to the earlier SI and cytokine level after treatment of the subject for Lyme disease. A decrease in one or both of SI and cytokine
level relative to the earlier SI and cytokine level can be indicative of a positive response to the Lyme disease treatment. No change or an increase in one or both of SI and cytokine level relative to the earlier SI and cytokine level can be indicative of no response to the various treatments. The composition can further comprise any one or both of: (a) a substantially purified polypeptide comprising an amino acid sequence having at least 80% sequence identity to any one of SEQ ID NOs:10-12, or to an antigenic fragment thereof; and (b) a substantially purified polypeptide comprising an amino acid sequence having at least 80% sequence identity to any one of SEQ ID NOs:13-18, or to an antigenic fragment thereof. The one or more cytokines can be selected from the group consisting of IL-1 beta, IL-4, IL-6, IL-8, IL-10, IL-13, MCP-1, G-CSF, IFN-gamma, and TNF-alpha. Determining the SI can comprise measuring uptake of tritiated thymidine, for example, by the lymphocytes or level of fluorescence. The determining the SI can comprise contacting the lymphocytes to IFN-α. The measuring one or more cytokines can comprise performing a bioassay, an immunoassay, flow cytometry, carboxyfluorscein succinimidyl (CFSE) ester staining, CD69 staining or radioimmunoassay (RIA). The immunoassay can be an enzyme-linked immunosorbent assay. The composition can further comprise one or more polypeptides derived from a species selected from the group consisting of *Borellia afzedii, Borellia garinii, Borellia miyamotoi, Babesia bovis, Babesia divergens, Babesia microti, Bartonella bacilliformis, Bartonella henselae, Bartonella quintana, Bartonella rochadimae, Anaplasma phagocytophilum, Ehrlichia ewingii, Ehrlichia chaffeenis, Ehrlichia canis, Neorickettsia sennetsu, Mycoplasma fermentans, Mycoplasma hominis, Mycoplasma pneumoniae, Mycoplasma genitalium, Mycopdasma penetrans, Rickettsia rickettsii*, and *Rickettsia typhis*.

Unless otherwise defined, all technical and scientific terms used herein have the same meaning as commonly understood by one of ordinary skill in the art to which this invention pertains.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

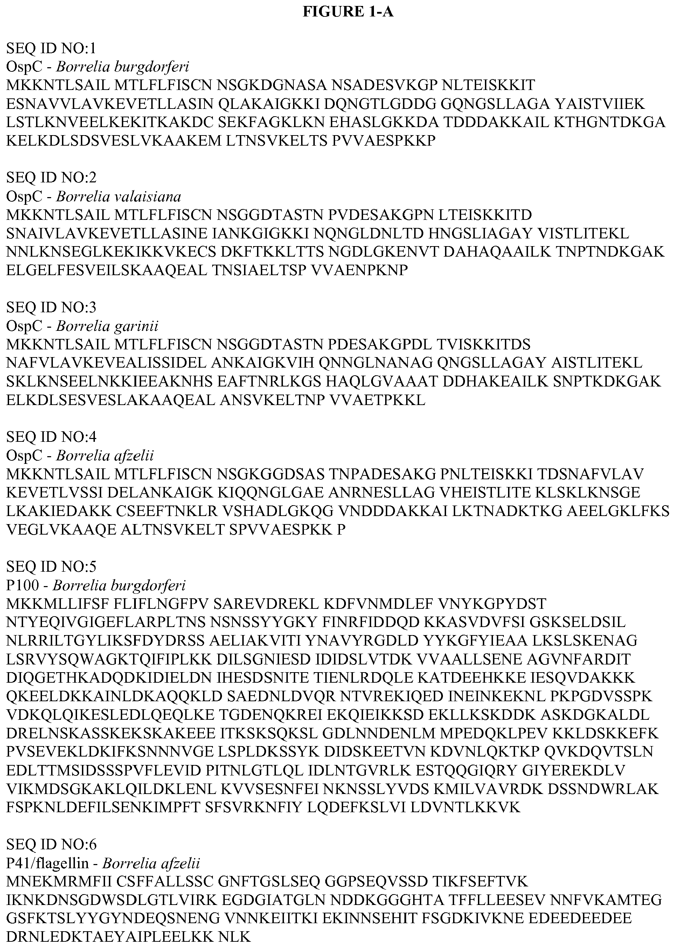

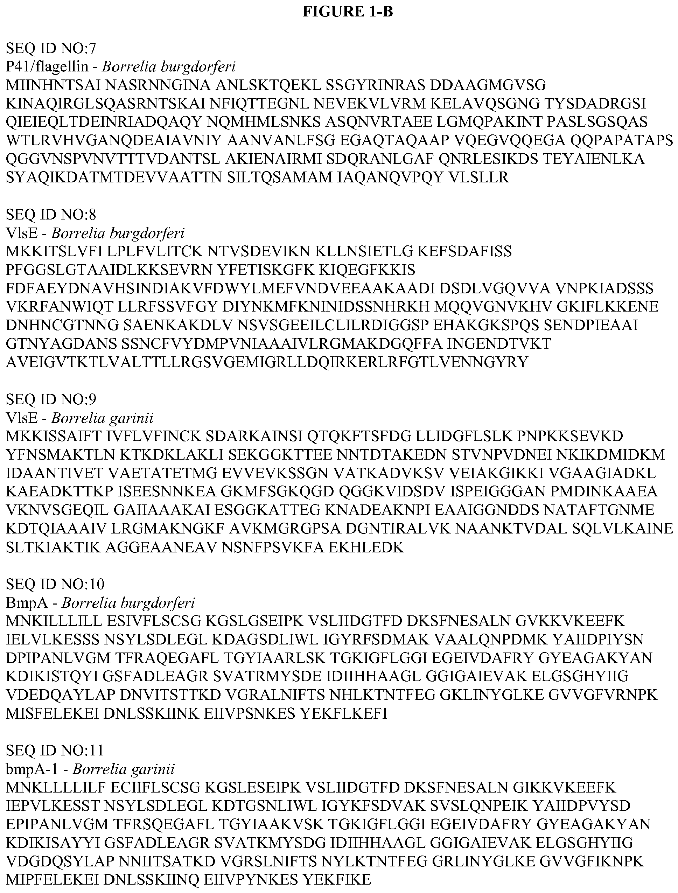

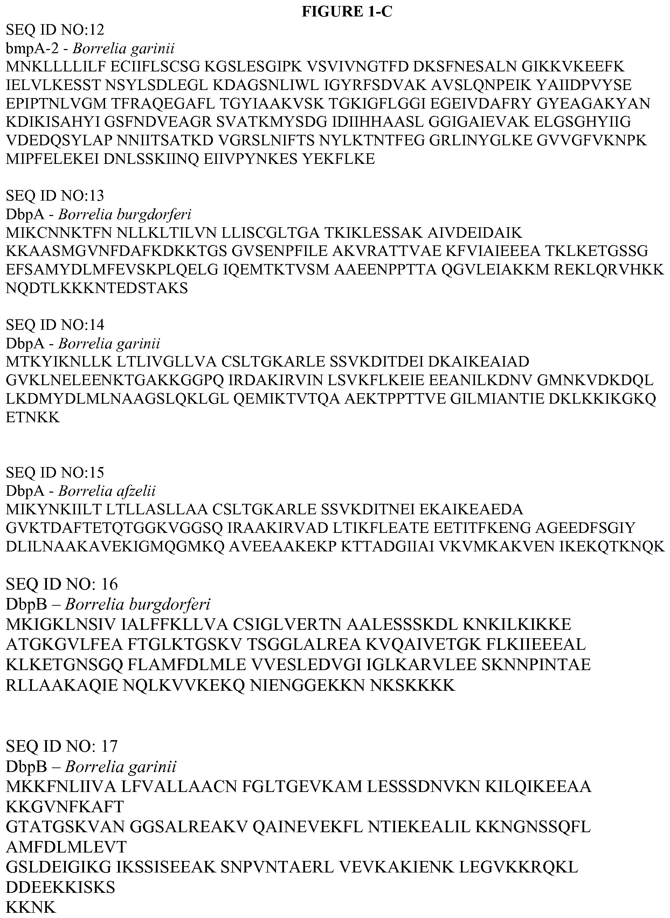

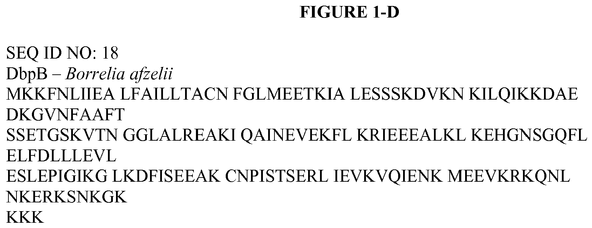

Figure 1 contains amino acid sequences for Borrelia antigens set forth as SEQ ID NOs:1-18.

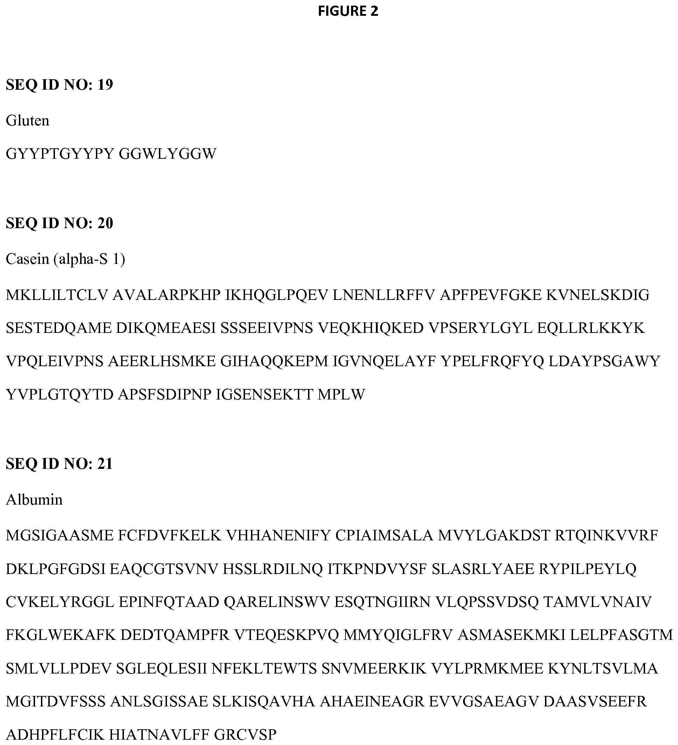

Figure 2 contains amino acid sequences for food antigens set forth as SEQ ID NOs: 19-21.

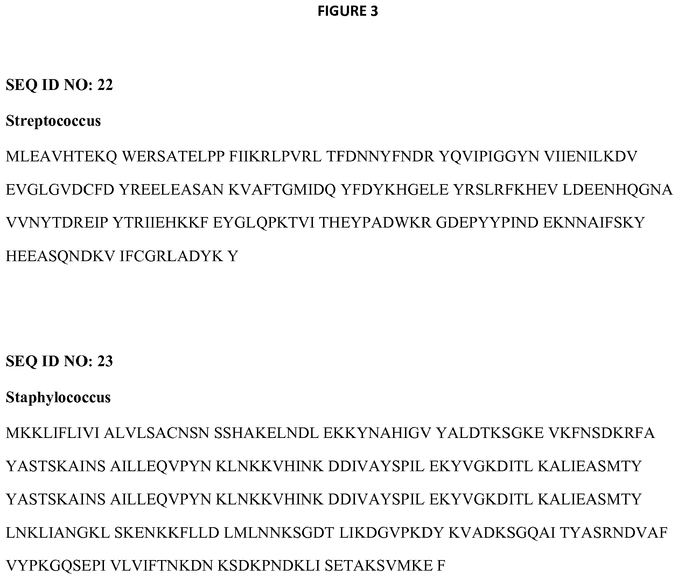

Figure 3 contains amino acid sequences for infectious agents set forth as SEQ ID NOs: 22-23.

### DETAILED DESCRIPTION

This document relates to methods and materials for diagnosing and monitoring treatment of Lyme disease. The present invention is in part based on the discovery that a composition comprising polypeptides derived from a *Borrelia* species can be used in a combined lymphocytic transformation test and cytokine production assay to detect Lyme disease in a subject with fewer false negative results compared to an antibody-based assay, such as a western blot, for example. Based at least in part on this discovery, compositions comprising polypeptides having at least 80% sequence identity to sequences encoding *Borrelia* polypeptides OspC, VlsE, DbpA, DbpB, p100, and p41, or to antigenic fragments thereof, and methods for using such compositions for detecting lymphocyte and cytokine proliferative responses are provided. Methods for diagnosing Lyme disease in a human subject are also provided.

### Compositions

This document provides methods and materials for preparing a composition comprising substantially pure polypeptides. A composition can include substantially purified polypeptide antigens, which are polypeptides having one or more immunoreactive epitopes. Antigens appropriate for the compositions provided herein can be selected based on the life-cycle or infection cycle of a bacteria such as, for example, *Borrelia.* Antigens can be polypeptides derived from or exhibiting sequence similarity to polypeptides from one or more of the following species of *Borrelia: Borrelia burgdorferi, Borrelia afzedii, Borrelia valaisiana,* or *Borrelia garinii.* Other species can include *Borrelia hermsii, Borrelia parkeri, Borrelia vincentii, Borrelia recurrentis, Borrelia miyamotoi, or Borrelia anserina*.

Compositions provided herein typically include more than one antigen, e.g., 2, 3, 4, 5, 6 or more antigens. In some cases, at least 6 polypeptide antigens are included. For example, the compositions provided herein can include polypeptides that include an amino acid sequence having 80% (e.g., 80%, 85%, 90%, 95%, 100%) or greater sequence identity to OspC, VlsE, DbpA, DbpB, p100, and p41 polypeptides, or to antigenic fragments thereof. The terms "polypeptide" and "protein" are used interchangeably herein and refer to any peptide-linked chain of amino acids, regardless of length or post- translational modification. A polypeptide for use in the materials and methods described herein can be an antigenic fragment of any of the polypeptides described herein, such as an antigenic fragment of an OspC, VlsE, DbpA, DbpB p100, or p41/flagellin polypeptide, provided the antigenic fragment includes at least one epitope of the reference polypeptide.

Antigens appropriate for the compositions provided herein can be substantially pure polypeptides. A substantially purified polypeptide or protein is substantially free of cellular material or other contaminating proteins from the cell or tissue source from which the protein is derived, or substantially free from chemical precursors or other chemicals when chemically synthesized. Any appropriate method for obtaining substantially pure polypeptides can be used. Also appropriate for the compositions provided herein can be whole proteins and peptides.

By way of example and without limitation, a polypeptide can be a native, recombinant, or chemically synthesized polypeptide or antigenic fragment thereof. In some cases, a polypeptide can be obtained from a whole organism lysate. In some cases, a polypeptide can be obtained by expression of a recombinant nucleic acid encoding the polypeptide or by chemical synthesis (e.g., by solid-phase synthesis or other methods well known in the art, including synthesis with an ABI peptide synthesizer; Applied Biosystems, Foster City, CA). Expression vectors that encode the polypeptide of interest can be used to produce a polypeptide. For example, standard recombinant technology using expression vectors encoding a polypeptide can be used.

Expression systems that can be used for small or large-scale production of the polypeptides provided herein include, without limitation, microorganisms such as bacteria transformed with recombinant bacteriophage DNA, plasmid DNA, or cosmid DNA expression vectors containing the nucleic acid molecules of the polypeptide of interest. The resulting polypeptides can be purified according to any appropriate protein purification method. In some cases, substantially pure polypeptides or antigenic fragments thereof can be purchased from a commercial supplier (e.g., Diarect, Freiburg, Germany).

As used herein, the term "percent sequence identity" refers to the degree of identity between any given query sequence and a subject sequence. Percentage of "sequence identity" is determined by comparing two optimally aligned sequences over a comparison window, where the fragment of the amino acid sequence in the comparison window may comprise additions or deletions (e.g., gaps or overhangs) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. The output is the percent identity of the subject sequence with respect to the query sequence. It is noted that a query nucleotide or amino acid sequence that aligns with a subject sequence can result in many different lengths, with each length having its own percent identity. Optimal alignment of sequences for comparison may be conducted by the local homology algorithm of Smith and Waterman (1981) Add. APL. Math. 2:482, by the homology alignment algorithm of Needleman and Wunsch (1970) J. Mol. Biol. 48:443, by the search for similarity method of Pearson and Lipman (1988) Proc. Natl. Acad. Sci. (USA) 85: 2444, by computerized implementations of algorithms such as GAP, BESTFIT, BLAST, PASTA, and TFASTA (Accelrys, Inc., 10188 Telesis Court, Suite 100 San Diego, Calif. 92121) or by inspection. Typically, the default values of 5.00 for gap weight and 0.30 for gap weight length are used.

The following Lyme antigens are provided to demonstrate the utility of the current testing platform. OspC is an outer surface protein expressed as the spirochete traverses to the mammalian host, whereas related outer surface polypeptides OspA and OspB are mainly expressed in the mid-gut of the tick. OspC proteins from *Borrelia burgdorferi, Borrelia valaisiana, Borrelia garinii*, and *Borrelia afzelii* are set forth in SEQ ID NOs: 1-4, respectively. An antigen for inclusion in a composition described herein can include an amino acid sequence having at least 80% (e.g., 80%, 85%, 90%, 95%, 100%) sequence identity to a *Borrelia* OspC polypeptide, or to an antigenic fragment thereof. In some cases, an antigen can include an amino acid sequence having at least 80% (e.g., 80%, 85%, 90%, 95%, 100%) identity to a polypeptide selected from SEQ ID NOs: 1-4, or to an antigenic fragment thereof.

VlsE is an outer surface lipoprotein that undergoes antigenic variation during disseminated infection. The *Borrelia* bacterium is hidden from the immune system by antigenic variation of surface proteins expressed by VlsE genes. Thus, antibodies to VlsE can serve as a diagnostic marker of later stages of *Borrelia* infection. VlsE proteins from *Borrelia burgdorferi* and *Borrelia garinii* are set forth in SEQ ID NOs:8-9, respectively. An antigen for inclusion in a composition described herein can include an amino acid sequence having at least 80% (e.g., 80%, 85%, 90%, 95%, 100%) sequence identity to a *Borrelia* VlsE polypeptide, or to an antigenic fragment thereof. In some cases, an antigen can include an amino acid sequence having at least 80% (e.g., 80%, 85%, 90%, 95%, 100%) identity to a polypeptide selected from SEQ ID NOs:8-9, or to an antigenic fragment thereof.

P100 is a high molecular weight major antigen of the membranous vesicle on the surface of *Borrelia burgdorferi* and is expressed late in *Borrelia* infection. Antibodies against p100 are usually of the IgG type and generally only appear in the chronic stage of the infection. P100 protein from *Borrelia burgdorferi* is set forth in SEQ ID NO:5. An antigen for inclusion in a composition described herein can include an amino acid sequence having at least 80% (e.g., 80%, 85%, 90%, 95%, 100%) sequence identity to a Borrelia p100 polypeptide, or to an antigenic fragment thereof. In some cases, an antigen can include an amino acid sequence having at least 80% (e.g., 80%, 85%, 90%, 95%, 100%) identity to polypeptide SEQ ID NO:5, or to an antigenic fragment thereof.

P41, or flagellin, is expressed in early and late *Borrelia* infection. P41/flagellin proteins from *Borrelia afzelii* and *Borrelia burgdorferi* are set forth in SEQ ID NOs:6-7. An antigen for inclusion in a composition described herein can include an amino acid sequence having at least 80% (e.g., 80%, 85%, 90%, 95%, 100%) sequence identity to a *Borrelia* p41/flagellin polypeptide, or to an antigenic fragment thereof. In some cases, an antigen can include an amino acid sequence having at least 80% (e.g., 80%, 85%, 90%, 95%, 100%) identity to a polypeptide selected from SEQ ID NOs:6-7, or to an antigenic fragment thereof.

Additional antigens appropriate for the compositions provided herein are bacterial antigens that bind host proteins. For example, BmpA is a *Borrelia* membrane protein that enhances spirochete colonization and survival in host tissues. BmpA and its three paralogous proteins, BmpB, BmpC, and BmpD, bind mammalian laminin. Accordingly, polypeptides suitable for the compositions provided herein can have an amino acid sequence with at least 80% (e.g., 80%, 85%, 90%, 95%, 100%) sequence identity to a *Borrelia* BmpA, BmpB, BmpC, or BmpD protein. In some cases, an antigen for inclusion in a composition described herein can include an amino acid sequence having at least 80% (e.g., 80%, 85%, 90%, 95%, 100%) sequence identity to BmpA amino acid sequences set forth as SEQ ID NO: 10 (*Borrelia burgdorferi*), SEQ ID NO:11 (*Borrelia garinii;* BmpA-1), or SEQ ID NO:12 (*Borrelia garinii;* BmpA-2).

Decorin-binding proteins A and B (DbpA and DbpB) bind decorin, a proteoglycan that associates with collagen. The decorin binding proteins promote binding of the spirochete to extracellular matrix proteins of host cells for maximum colonization of host tissues including skin and joints. Accordingly, polypeptides suitable for the compositions provided herein can have an amino acid sequence with at least 80% (e.g., 80%, 85%, 90%, 95%, 100%) sequence identity to a *Borrelia* DbpA. In some cases, an antigen for inclusion in a composition described herein can include an amino acid sequence having at least 80% (e.g., 80%, 85%, 90%, 95%, 100%) sequence identity to DbpA amino acid sequences set forth as SEQ ID NO: 13 (*Borrelia burgdorferi*), SEQ ID NO: 14 (*Borrelia garinii*), or SEQ ID NO: 15 (*Borrelia afzelii*). In some cases, an
antigen for inclusion in a composition described herein can include an amino acid sequence having at least 80% (e.g., 80%, 85%, 90%, 95%, 100%) sequence identity to DbpB amino acid sequences set forth as SEQ ID NO: 16 (*Borrelia burgdorferi*), SEQ ID NO: 17 (*Borrelia garinii*), or SEQ ID NO: 18 (*Borrelia afzedii*)

Other host receptor binding proteins can include P66, a 66-kilodalton (kD) spirochetal polypeptide that binds platelet-specific integrin α2bβ3 and the vitronectin receptor αvβ3; Bgp, a 26-kD glycosaminoglycan-binding polypeptide that binds heparin sulfate and dermatin sulphate; and Fbp, a 47 kD fibronectin-binding polypeptide. Accordingly, polypeptides exhibiting at least 80% (e.g., 80%, 85%, 90%, 95%, 100%) sequence identity to *Borrelia* P66, Bgp, or Fbp polypeptides are also suitable for inclusion.

In some cases, a composition can include, or consist essentially of, (a) a substantially purified polypeptide comprising an amino acid sequence having at least 80% sequence identity to any one of SEQ ID NOs: 1-4, or to an antigenic fragment thereof; (b) a substantially purified polypeptide comprising an amino acid sequence having at least 80% sequence identity to SEQ ID NO:5, or to an antigenic fragment thereof; (c) a substantially purified polypeptide comprising an amino acid sequence having at least 80% sequence identity to any one of SEQ ID NOs:6-7, or to an antigenic fragment thereof; and (d) a substantially purified polypeptide comprising an amino acid sequence having at least 80% sequence identity to any one of SEQ ID NOs:8-9, or to an antigenic fragment thereof.

A composition provided herein can include, or consist essentially of, (a) a substantially purified polypeptide comprising SEQ ID NO:1; (b) a substantially purified polypeptide comprising SEQ ID NO:5; (c) a substantially purified polypeptide comprising SEQ ID NO:7; and (d) a substantially purified polypeptide comprising SEQ ID NO:8.

A composition provided herein can additionally include (a) a substantially purified polypeptide comprising an amino acid sequence having at least 80% (e.g., 80%, 85%, 90%, 95%, 100%) sequence identity to any one of SEQ ID NOs: 10-12, or to an antigenic fragment thereof; and/or (b) a substantially purified polypeptide comprising an amino acid sequence having at least 80% (e.g., 80%, 85%, 90%, 95%, 100%) sequence identity to any one of SEQ ID NOs:13-18, or to an antigenic fragment thereof.

Concurrent infections of Lyme disease and other tick-borne illnesses can occur. Thus, a composition provided herein can include one or more antigens derived from or exhibiting sequence similarity to one or more tick-borne infectious agents. For example, a composition can include one or more polypeptides derived from a species of the protozoan parasite *Babesia* (e.g., *Babesia bovis, Babesia divergens, Babesia microti)*. A composition can include one or more polypeptides derived from a species of the Gramnegative bacterium *Bartonella* (e.g., *Bartonella bacilliformis, Bartonella henselae, Bartonella quintana, Bartonella rochalimae)*, from a species of the *rickettsiales* bacteria genus *Anaplasma* (e.g., *Anaplasma phagocytophilum)* or the genus *Ehrlichia* (e.g., *Ehrlichia ewingii, Ehrlichia chaffeenis, Ehrlichia canis, Neorickettsia sennetsu),* from a species of mycoplasma bacteria (e.g., *Mycoplasma fermentans, Mycoplasma hominis, Mycoplasma pneumoniae, Mycoplasma genitalium, Mycoplasma penetrans)*, or from a species of *Rickettsia* (e.g., *Rickettsia rickettsii, Rickettsia typhi)* and others.

In some cases, a composition provided herein can further include Interferon-alpha (IFN-α). The addition of IFN-α to a composition provided herein can suppress non-specific "bystander" cell proliferation when the composition is contacted to cells in vitro. See Bieger et al., EP1058116 A2; von Baehr et al., J. Immunological Methods 251:63-71 (2001). IFN-α can be a native, recombinant, or chemically synthesized polypeptide. IFN-α also can be purchased from commercial suppliers such as R&D Systems (Minneapolis, MN).

### Methods for Using Compositions

Also provided herein are methods for diagnosing a *Borrelia* infection. In some cases, the methods for diagnosing can include determining a stimulation index (SI). As used herein, "stimulation index" refers to the ratio of measured proliferation of lymphocytes and antigen-presenting cells from an individual when exposed to antigen, to the measured proliferation of said cells in the absence of antigen. In some cases, a stimulation index can be determined by measuring ³H-thymidine uptake in peripheral mononuclear cells. For example, stimulation indices for responses by lymphocytes to compositions provided herein can be calculated as the maximum counts per minute (CPM) in response to a composition divided by the control CPM (for un-contacted cells). A stimulation index (SI) equal to or greater than about two times (e.g., about 1.7, 1.8, 1.9, 2, 2.1, 2.2, 2.3, 2.4, 2.5, 2.6, 2.7, 2.8, 2.9 or 3 times) the level determined for a control can be considered "positive" for sensitivity or immunoreactivity to the antigen tested. A SI equal to or greater than about 10 can be considered "strongly positive" for sensitivity or immunoreactivity to the antigen tested.

In some cases, determining a stimulation index can include an immune tolerance test (ITT). Lymphocytes, including B-cells and T-cells play a large role in the immune response system. B-cells play a major role in the humoral immune response, while T- cells are intimately involved with cell-mediated immune responses. ITT measures lymphocyte proliferation and can determine an increase in cell number, cell growth, cell division, or cell expansion in response to contact with an immunoreactive compound or composition. For example, immunoreactivity of lymphocytes to a composition of substantially purified polypeptides derived from, for example, *Borrelia burgdorferi* can be detected. Increased immunoreactivity of lymphocytes to such substantially purified polypeptides can indicate sensitivity of the subject to Lyme antigen.

In some cases, determining a SI using ITT can include contacting a composition provided herein to lymphocytes in vitro. Contacted lymphocytes can be collected and the amount of cell proliferation can be measured by any appropriate method. For example, a common assay for T cell proliferation entails measuring tritiated thymidine incorporation into the DNA of dividing cells. In some cases, therefore, lymphocytes are additionally cultured in a medium containing a means of detecting cellular proliferation (e.g., a radiolabeled nucleoside such as ³H-thymidine). The proliferation of T cells can be measured in vitro by determining the amount of ³H-labeled thymidine, for example, incorporated into the replicating DNA of cultured cells. Tritiated thymidine incorporation can provide a quantitative measure of the rate of DNA synthesis, which is typically directly proportional to the rate of cell division. The amount of tritiated thymidine incorporated can be measured by liquid scintillation counting. Other methods of measuring lymphocyte proliferation can include, without limitation, detecting and measuring cells on the basis of the expression of various cell surface markers (e.g.,
CD69), fluorescence-based methods, cell division tracking dyes (e.g., carboxyfluorescein succinimidyl ester (CFSE)), CD69 staining and flow cytometry.

In some cases, non-specific cell proliferation can be suppressed by contacting lymphocytes to a composition provided herein in the presence of interferon alpha (IFN-α). The addition of IFN-α to cultured cells can suppress non-specific "bystander" proliferation while improving antigen-specific T-cell proliferation. See Bieger et al., EP1058116 A2; von Baehr et al., J. Immunological Methods 251:63-71 (2001). IFN-α can be present in a composition provided herein, or can be directly contacted to lymphocytes in culture.

The ITT can be performed in conjunction with a cytokine assay. Cytokines are the chemical messengers of the immune system and serve as markers for inflammatory processes. An encounter with physical stimuli (i.e. bacterial, viral, fungal, parasite infection, toxins, food, allergens, auto antigens, or medications) challenges the immune system. In response to such stimuli, immune cells secrete cytokines as a primary defense mechanism. Elevated cytokine levels may be an indication of an active immune response to physical stimuli. Cytokine assays for determining immunological responsiveness generally involve measuring cytokine or growth factor production. ITT and cytokine assays can be performed using peripheral blood mononuclear cells (PBMCs). PBMCs can be obtained from a subject's whole blood. Suitable methods for obtaining PBMCs can be used. For example, a whole peripheral blood sample can be obtained from a subject having or suspected of having Lyme disease (e.g., experiencing symptoms associated with Lyme disease). The PBMCs, which include lymphocytes, macrophages, and other white blood cells, can be isolated from whole peripheral blood by any appropriate method (e.g., centrifugation or density gradient). In some cases, PBMCs can be isolated from the whole blood by centrifugation, washed, and then suspended in medium with antibiotic, e.g., RPMI medium (Gibco, Grand Island, N.Y.) with penicillin/streptomycin and 1% glutamine. Human serum may also be added to samples, typically to a final concentration of about 5%. PBMCs isolated from a subject's blood sample can be split into two portions: one portion to be used for ITT, and a second portion to be used for cytokine assays. In some cases, therefore, a method for diagnosing Lyme disease in a subject can include the steps of: (a) isolating PBMCs from the subject;
(b) incubating a portion of the PBMCs with a composition provided herein or a control antigen (e.g., pokeweed mitogen); (c) determining a stimulation index of PBMCs; and (d) comparing the stimulation index from step (c) with a stimulation index determined for a control.

In conjunction with ITT, a portion of the PBMCs obtained from a subject can be used for a cytokine assay. Cells can be cultured alone or in the presence of a composition provided herein (e.g., *Borrelia* antigens) or a control antigen (e.g., phytohemagglutinin). Cell-free supernatants can be collected from stimulated and non-stimulated or control cell cultures for cytokine analysis. Among the factors that can be measured using cytokine assays are: any of the interleukins, tumor necrosis factors, interferons, colony stimulating factors, leukemia inhibitory factor, transforming growth factors, or epidermal growth factor. In some cases, levels of one or more cytokines such as Interleukin 1 beta (IL-1 beta), IL-6, IL-8, IL-10, IL-13, MCP-1, G-CSF, Interferon- gamma (IFN-γ), and Tumor Necrosis Factor-alpha (TNF-α), can be measured. Methods of measuring one or more cytokine levels can include, for example, a bioassay, an immunoassay, a radioimmunoassay (RIA), an enzyme-linked immunosorbent assay (ELISA), or measurement of messenger RNA levels. In general, immunoassays involve using a monoclonal antibody to the cytokine of interest to specifically bind and detect the cytokine. Immunoassays are well-known in the art and can include both competitive assays and immunometric assays (see generally Ausubel et al., Current Protocols in Molecular Biology, 11.2.1-11.2.19 (1993); Laboratory Techniques in Biochemistry and Molecular Biology, Work et al., ed. (1978)).

In some cases, the level of one or more cytokines can be measured if a subject's stimulation index is increased relative to a control stimulation index (e.g., a SI determined for a control subject not having Lyme disease or symptoms associated with Lyme disease). For example, a method for diagnosing Lyme disease in a subject can include (a) determining a stimulation index (SI) of the lymphocytes, where determining the SI include performing a lymphocyte proliferation assay by contacting a composition provided herein to lymphocytes obtained from the subject; and (b) measuring one or more cytokines in a sample from the subject, wherein an increase in the SI and/or an increase the level of a cytokine relative to a control can be indicative of Lyme disease in the subject. A stimulation index of around example 0.5, 0.6 to 1.5) and/or cytokine levels comparable to control can be indicative of an absence of Lyme disease in the subject.

In some cases, the level of one or more cytokines can be measured regardless of whether the subject's stimulation index is increased relative to the control. In this manner, the measurement of one or more cytokine levels when a stimulation index is increased relative to the control will provide additional diagnostic information. Generally, a positive ITT response to a composition provided herein relative to a control is not previously described and is not, by itself, diagnostic of an on-going immune response to *Borrelia.* A positive ITT can be determined several months or even years after the initial exposure to one or more *Borrelia* antigens. Measurement of cytokines is required in order to arrive at a confirmed current immune response to *Borrelia.*
Elevations in cytokines are indicative of an acute infection or recent exposure to one or more antigens present in the composition assayed. An increase in the pro-inflammatory cytokines IL- 1β, IL-6, IL-8, IL-13, TNF-α, and IFN-γ suggests an increased inflammatory response which can contribute to (or are in response to) symptoms associated with Lyme disease.

### Methods of Monitoring Treatment of Lyme Disease

Also provided herein are methods of monitoring a subject's response to Lyme disease treatment. As used herein, the term "treat" or "treatment" is defined as the application or administration of a treatment regimen, e.g., a therapeutic agent or modality, to a subject, e.g., a patient. The subject can be a patient having Lyme disease or a symptom of Lyme disease, or at risk of developing Lyme disease (e.g., frequently outdoors, living in a tick infested area). The treatment can be to cure, heal, alleviate, relieve, alter, remedy, ameliorate, palliate, improve or affect Lyme disease or symptoms associated with Lyme disease. For example, the methods provided herein can be used to monitor a subject's response to standard therapeutic regimen for Lyme disease, which can include, without limitation, oral administration of antibiotics (e.g., doxycycline, amoxicillin, or cefuroxime axetil) and intravenous administration of medicaments such as ceftriaxone and penicillin. Some subjects treated with antibiotics in the early stages of infection can recover completely. Some patients, particularly those diagnosed with later stages of disease, may have persistent or recurrent symptoms. For such subjects, Lyme disease can be treated with extended antibiotic therapy (e.g., one, two, three, four, or more weeks of antibiotic treatment). In some cases, the methods provided herein also can be used to monitor a subject's response to standard therapeutic regimen for a co-infection of *Babesia, Bartonella, Ehrlichia, Anaplasma*, and/or *Rickettsia*. Standard therapeutic regimens for *Babesia* can include administration of medicaments such as atovaquone (Mepron) plus azithromycin (Zithromax), clindamycin and oral quinine. Standard therapeutic regimens for *Bartonella* can include administration of medicaments such as erythromycin, fluoroquinolone, or rifampin. *Ehrlichia* is frequently treated with the administration of medicaments such as doxycycline and rifampin.

In some cases, the methods can be used to monitor a subject's response to Lyme disease treatment by, for example, determining a stimulation index and level of one or more cytokines at multiple time-points. The subject can be monitored in one or more of the following periods: prior to beginning of treatment; during the treatment; or after one or more elements of the treatment have been administered. For example, the methods provided herein (e.g., ITT and cytokine assays) can be performed at predetermined time- point before or while a subject is treated with antibiotics. The methods provided herein can be repeated at one or additional later time- points to observe any change in stimulation index and/or levels of one or more cytokines over time. In some cases, stimulation index and cytokine levels can be determined prior to a subject receiving
treatment to establish a baseline from which a health care professional or researcher can observe a positive or negative response to a particular treatment regimen. A positive response can be indicated by a decrease in stimulation index and/or cytokine levels relative to an earlier stimulation index and cytokine level. A negative response to treatment can be indicated by an increase in stimulation index and/or increase in cytokine levels relative to a baseline stimulation index and cytokine level. If no changes in stimulation index and/or cytokine levels relative to a baseline measurement are detected, the Lyme disease treatment regimen may be ineffective for the subject. Monitoring can be used to evaluate the need for further treatment with the same or a different therapeutic agent or modality. Generally, a decrease in one or more of the parameters described above is indicative of the improved condition of the subject.

In some cases, the methods provided herein can be used in parallel with other methods of monitoring Lyme disease treatment, including subjective (e.g., self-report of symptoms) and objective measurements of Lyme disease symptoms. For example, the methods provided herein can be used in parallel with clinical observations of, or a subject's self-reporting of, pain, fever, headache, swelling, or other symptoms associated with Lyme disease. In some cases, the methods provided herein can be used in parallel with Western blot analysis or serological assays for the presence of *Borrelia-specific* antibodies.

### Articles of Manufacture

Also provided herein are articles of manufacture including one or more of the compositions provided herein. Instructions for use can include instructions for diagnostic applications of the compositions for diagnosing Lyme disease and/or monitoring the response of a subject to treatment of Lyme disease. The kit can include one or more other elements including: instructions for use; and other reagents such as IFN-α, ELISA reagents, and one or more reagents for detecting lymphocyte proliferation.

Kits as provided herein can also include a mailer (e.g., a postage paid envelope or mailing pack) that can be used to return the sample for analysis, e.g., to a laboratory. The kit can include one or more containers for the sample, or the sample can be in a standard blood collection vial. The kit can also include one or more of an informed consent form, a test requisition form, and instructions on how to use the kit in a method described herein. Methods for using such kits are also included herein. One or more of the forms (e.g., the test requisition form) and the container holding the sample can be coded, for example, with a bar code for identifying the subject who provided the sample.

### EXAMPLES

### Example 1 - Methods of Cell and Reagent Preparation

### Cell Preparation

Sixteen milliliters (mL) of Lymphoprep™ solution (Axis-Shield, Oslo, Norway) is spun down in a greiner tube at 2800 rpm (1825 g) for 1-2 minutes at room temperature. Any fluid that is above the filter is removed. Any unused tubes are kept at 4°C until use. Tubes can be warmed in a waterbath or incubator before use.

Blood should be collected in 3.2% citric acid and kept at room temperature. After tube warm-up, add up to 16 mL whole blood to each tube by pouring the blood from two vacutainers. The tubes are centrifuged at 2800 rpm (1825 g) for 15 minutes at room temperature. Some serum is removed and then the tube is swirled to mix the buffy layer and loosen cells from the tube walls. The buffy layer is poured into a fresh 50 mL tube. The tube is filled with Dulbecco's Phosphate Buffered Saline (PBS), or with HBSS without calcium and magnesium. Buffers should be at pH 7.0. The tubes are spun at 1600 rpm (596 g) for 10 minutes at room temperature. The buffer is removed, and the pellet is vortexed. The buffer is poured off and the cells are vortexed again and spun at 1000 rpm (233 g) for ten minutes at room temperature. A mixture of media is added:
50% RPMI 1640 + 50% RPMI 1640 containing 20% Hepes plus 800 µL glutamine per 50 mL of medium plus 50 µL gentamycin per 100 mL medium. All tubes from one patient are combined for a final volume of 4 mL. A bulb pipette can be used.

A tissue culture is prepared with 1 mL human AB positive (or 80% positive, 20% negative) serum. The serum is heat inactivated at 56°C for one hour before use. The cells (4 mL) are added to the T-flask and incubated for 30-45 minutes. After incubation, cells in the T- flask are "washed" with the medium inside, and all fluid is removed to a fresh 50 mL tube. The T-flask is rinsed with medium mix without human serum added until 10 mL remains in the flask (human serum concentration is now 10%). An aliquot of cells is removed for counting. 50 µL cells are added to 450 µL Trypan blue, mixed, and counted on a hemocytometer. The number of cells in the 10 mL is calculated. The cells are diluted with medium with 10% human serum to a ratio of approximately 800,000 - 1,000,000 cells per mL.

### Preparation of IFN-alpha Solution

Prepare sterile PBS with 10% v/v glycerol. Reconstitute the vials of IFN-alpha (having 350,000,000 units per vial) with 2 mL of the sterile PBS/glycerol solution. The concentration of the solution (Solution 1) is now 175,000,000 units per mL or 175,000 units per µL. Freeze aliquots of the solution at -20 degrees C.

Take 50 µL of Solution 1 (8,750,000 units) and dilute to 5000 µL by adding 4950 µL PBS/glycerol solution. The concentration of the solution (Solution 2) is now 1,750 units per µL. Aliquot this solution 2 in 500 µL volumes and freeze at -20 degrees C. To prepare the alpha IFN for use in wells: take 100 µL of solution 2 (175,000 units/100 µL) and add to 1200 µL sterile PBS sans glycerol. This solution (Solution 3) now has 135 units per µL. Add 50 µL of solution 3 (equals 6750 units) to each well on the plate.

### Example 2 - Immune Tolerance Test

Culture plates are precoated with recombinant *Borrelia* antigens in 50 µL/well medium without human serum. The coated plates are wrapped in parafilm and stored at - 20 C until use.

To all wells add 50 µL of prepared solution. The IFN-alpha concentration should be 6250-7500 Units per well. PBMCs (1 x 10 in 1 mL of 10% medium) are pipetted into the 24-well cell culture plate pre-coated with *Borrelia* antigens in single dilutions, and incubated at 37 C with 5% CO. One negative control (lymphocytes in 10% medium without antigen) and one positive control (lymphocytes in 10% medium plus 2 µg/mL pokeweed mitogen) are included on each plate. On cell culture day 5, the culture is pulsed for 5 hours with 3 µCi 25 methyl-3H-thymidine (30 µL/well of a 1:10 dilution (with PBS) of stock Thymidine, 5 mCi/5mL) and the radioactivity is measured in a liquid scintillation counter. Cell proliferation is recorded as counts per minute (cpm) which are converted to a stimulation index (SI) representing the cpm in the test well divided by the cpm in the negative control well.

### Example 3 - Cytokine Assay

Venous blood is obtained from healthy individuals (the control group) or patients in yellow top tubes (ACD tubes). PBMCs are isolated from peripheral blood using commercially available Ficoll-Hypaque density gradients, washed twice with phosphate-buffered saline, and resuspended in complete culture medium at a concentration of 1 x 106 cells per mL. Cells are cultured alone, with *Borrelia* antigens, or with 5 µg/mL phytohemagglutinin (PHA) at 37 C and 5% CO2. The cell-free supernatants are collected after 24 hours by centrifugation, divided into aliquots and stored at -80 C until use.

Following the cell cultures, stimulated and non-stimulated PBMCs supernatants are then assayed using the 96-well Bio-Plex suspension array system, which utilizes xMAP detection technology (Bio-Rad Corporation, Hercules, CA). The cytokines measured are IL-1β, IL-6, IL- 8, IL-10, IL-13, MCP-1, G-CSF, IFN-γ, and TNF-α. The principle of these 96-well plate-formatted magnetic bead-based assays is similar to a capture sandwich immunoassay. All reagents, working standards, and samples are prepared as per the manufacturer's specifications and run in duplicate. Samples are read using a Bio-Plex reader. Spontaneous and stimulated levels of cytokines are determined using the Bio-Plex Manager Software (Bio-Rad), interpolating from the standard curve (Fogistic-5PF curve fit). Data from the reaction are then acquired using the Bio-Plex suspension array system, a dual-laser flow-based microplate reader system. Intensity of fluorescence detected on the beads indicates the relative quantity of targeted molecules.
A high speed digital processor efficiently manages the data output, which further analyzes and presents as fluorescence intensity on Bio-Plex Manager™ software (Bio- Rad). Controls are included on all plates. Inter- and intra-assay coefficients of variability were less than 20%. Calibrations of the instruments are performed before each assay and validation of the instruments is performed following calibration. The unstimulated and the stimulated results are reported in units of pg/mL.

## Claims

1. A method for diagnosing Lyme disease in a subject, the method comprising:
a. determining a stimulation index (SI) of lymphocytes obtained from the subject, wherein said determining comprises contacting a composition to the lymphocytes, and wherein the composition comprises:
(i) a substantially purified polypeptide comprising an amino acid sequence having at least 80% sequence identity to any one of SEQ ID Nos. 1-4, or to an antigenic fragment thereof;
(ii) a substantially purified polypeptide comprising an amino acid sequence having at least 80% sequence identity to SEQ ID NO:5, or to an antigenic fragment thereof;
(iii) a substantially purified polypeptide comprising an amino acid sequence having at least 80% sequence identity to any one of SEQ ID Nos. 6-7, or to an antigenic fragment thereof; and
(iv) a substantially purified polypeptide comprising an amino acid sequence having at least 80% sequence identity to any one of SEQ ID Nos. 8-9, or to an antigenic fragment thereof; and
b. measuring one or more cytokines in a sample from the subject, wherein an increase in one or both of SI and cytokine level relative to a control is indicative of Lyme disease in the subject.

2. The method of claim 1, wherein the composition further comprises any one or both of: (a) a substantially purified polypeptide comprising an amino acid sequence having at least 80% sequence identity to any one of SEQ ID Nos:10-12, or to an antigenic fragment thereof; and
(b) a substantially purified polypeptide comprising an amino acid sequence having at least 80% sequence identity to any one of SEQ ID Nos:13-15, or to any one of SEQ ID Nos: 16-18, or to an antigenic fragment thereof.

3. The method of claim 1, wherein the one or more cytokines are selected from the group consisting of IL-1 beta, IL-6, IL-8, IL-10, IL-13, MCP-1, G-CSF, IFN-gamma, and TNF-alpha.

4. The method of claim 1, wherein said determining a stimulation index comprises measuring uptake of tritiated thymidine by the lymphocytes.

5. The method of claim 1 wherein said determining a stimulation index comprises contacting the lymphocytes to IFN-α.

6. The method of claim 1, wherein measuring one or more cytokines comprises performing a bioassay, an immunoassay, flow cytometry, or radioimmunoassay (RIA).

7. The method of claim 6, wherein the immunoassay is an enzyme-linked immunosorbent assay.

8. The method of claim 1, wherein the composition further comprises one or more polypeptides derived from a species selected from the group consisting of *Babesia bovis, Babesia divergens, Babesia microti, Bartonella bacilliformis, Bartonella henselae, Bartonella quintana, Bartonella rochalimae, Anaplasma phagocytophilum, Ehrlichia ewingii, Ehrlichia chaffeenis, Ehrlichia canis, Neorickettsia sennetsu, Mycoplasma fermentans, Mycoplasma hominis, Mycoplasma pneumoniae, Mycoplasma genitalium, Mycoplasma penetrans, Rickettsia rickettsii*, and *Rickettsia typhi*.

9. A method for determining the likelihood of developing symptoms associated with Lyme disease in a subject, the method comprising:
a. determining a stimulation index (SI) of lymphocytes obtained from the subject, wherein said determining comprises contacting a composition to the lymphocytes, wherein the composition comprises:
(i) a substantially purified polypeptide comprising an amino acid sequence having at least 80% sequence identity to any one of SEQ ID Nos. 1-4, or to an antigenic fragment thereof;
(ii) a substantially purified polypeptide comprising an amino acid sequence having at least 80% sequence identity to SEQ ID NO:5, or to an antigenic fragment thereof;
(iii) a substantially purified polypeptide comprising an amino acid sequence having at least 80% sequence identity to any one of SEQ ID Nos. 6-7, or to an antigenic fragment thereof; and
(iv) a substantially purified polypeptide comprising an amino acid sequence having at least 80% sequence identity to any one of SEQ ID Nos. 8-9, or to an antigenic fragment thereof; and
b. measuring one or more cytokines in a sample from the subject, wherein an increase in one or both of SI and cytokine level relative to a control is indicative of an increased likelihood of developing symptoms associated with Lyme disease in the subject.

10. A method of monitoring treatment of Lyme disease in a subject, the method comprising
a. determining a baseline stimulation index (SI) of lymphocytes obtained from the subject, wherein said determining comprises contacting a composition to the lymphocytes, wherein the composition comprises:
(i) a substantially purified polypeptide comprising an amino acid sequence having at least 80% sequence identity to any one of SEQ ID Nos: 1-4, or to an antigenic fragment thereof;
(ii) a substantially purified polypeptide comprising an amino acid sequence having at least 80% sequence identity to SEQ ID NO:5, or to an antigenic fragment thereof;
(iii) a substantially purified polypeptide comprising an amino acid sequence having at least 80% sequence identity to any one of SEQ ID Nos:6-7, or to an antigenic fragment thereof; and
(iv) a substantially purified polypeptide comprising an amino acid sequence having at least 80% sequence identity to any one of SEQ ID Nos:8-9, or to an antigenic fragment thereof;
b. measuring one or more cytokines in a sample from the subject; and
c. comparing a later SI and cytokine level to the earlier SI and cytokine level after treatment of the subject for Lyme disease, wherein a decreasing one or both of SI and cytokine level relative to the earlier SI and cytokine level is indicative of a positive response to the Lyme disease treatment, and wherein no change or an increase in one or both of SI and cytokine level relative to the earlier SI and cytokine level is indicative of no response to the Lyme disease treatment.

11. A composition for use in a method of any one of claims 1 to 10,
the composition comprising:
(a) a substantially purified polypeptide comprising an amino acid sequence having at least 80% sequence identity to any one of SEQ ID Nos. 1-4, or to an antigenic fragment thereof;
(b) a substantially purified polypeptide comprising an amino acid sequence having at least 80% sequence identity to SEQ ID NO:5, or to an antigenic fragment thereof;
(c) a substantially purified polypeptide comprising an amino acid sequence having at least 80% sequence identity to any one of SEQ ID . 6-7, or to an antigenic fragment thereof; and
(d) a substantially purified polypeptide comprising an amino acid sequence having at least 80% sequence identity to any one of SEQ ID Nos. 8-9, or to an antigenic fragment thereof

12. The composition of claim 11 comprising:
(a) a substantially purified polypeptide comprising SEQ ID NO:1; (b) a substantially purified polypeptide comprising SEQ ID NO:5;
(c) a substantially purified polypeptide comprising SEQ ID NO:7; and
(d) a substantially purified polypeptide comprising SEQ ID NO:8.

13. The composition of any one of claims 11 or 12, wherein the composition further comprises any one or both of:
(a) a substantially purified polypeptide comprising an amino acid sequence having at least 80% sequence identity to any one of SEQ ID Nos:10-12, or to an antigenic fragment thereof; and
(b) a substantially purified polypeptide comprising an amino acid sequence having at least 80% sequence identity to any one of SEQ ID Nos:13-15, or any one of SEQ ID Nos:16-18, or to an antigenic fragment thereof.

14. The composition of any one of claims 11, or 12, wherein the composition further comprises Interferon-alpha (IFN-α).

15. The composition of any one of claims 11, or 12, wherein the composition further camprises one or more polypeptides derived from a species selected from the group consisting of *Babesia bovis, Babesia divergens, Babesia microti, Bartonella bacilliformis, Bartonella henselae, Bartonella quintana, Bartonella rochalimae, Anaplasma phagocytophilum, Ehrlichia ewingii, Ehrlichia chaffeenis, Ehrlichia canis, Neorickettsia sennetsu, Mycoplasmafermentans, Mycoplasma hominis, Mycoplasma pneumoniae, Mycoplasma genitalium, Mycoplasma penetrans, Rickettsia rickettsii*, and *Rickettsia typhi*.

## Patentansprüche

1. Eine Verfahren zur Diagnose von Zeckenborreliose in einem Testsubjekt, das Verfahren umfassend:
a. Bestimmung eines Stimulationsindexes (SI) von Lymphozyten erhalten von dem Testsubjekt, wobei die Bestimmung das in Kontakt bringen einer Zusammensetzung mit den Lymphozyten umfasst, und wobei die Zusammensetzung umfasst:
(i) ein im Wesentlichen gereinigtes Polypeptid umfassend eine Aminosäuresequenz mit mindestens 80% Sequenzidentität zu einer der SEQ ID Nos. 1-4, oder zu einem antigenen Fragment davon;
(ii) ein im Wesentlichen gereinigtes Polypeptid umfassend eine Aminosäuresequenz mit mindestens 80% Sequenzidentität zu SEQ ID No. 5, oder zu einem antigenen Fragment davon;
(iii) ein im Wesentlichen gereinigtes Polypeptid umfassend eine Aminosäuresequenz mit mindestens 80% Sequenzidentität zu einer der SEQ ID Nos. 6-7, oder zu einem antigenen Fragment davon;
(iv) ein im Wesentlichen gereinigtes Polypeptid umfassend eine Aminosäuresequenz mit mindestens 80% Sequenzidentität zu einer der SEQ ID Nos. 8-9, oder zu einem antigenen Fragment davon; und
b. Messen eines oder mehrerer Cytokine in einer Probe des Testsubjekts, wobei ein Anstieg in einem SI oder Cytokin-Gehalt oder beiden relativ zu einer Kontrolle für Zeckenborreliose in dem Testsubjekt indikativ ist.

2. Das Verfahren nach Anspruch 1, wobei die Zusammensetzung des Weiteren umfasst eine oder beide von:
(a) ein im Wesentlichen gereinigtes Polypeptid umfassend eine Aminosäuresequenz mit mindestens 80% Sequenzidentität zu einer der SEQ ID Nos. 10-12, oder zu einem antigenen Fragment davon; und
(b) ein im Wesentlichen gereinigtes Polypeptid umfassend eine Aminosäuresequenz mit mindestens 80% Sequenzidentität zu einer der SEQ ID Nos. 13-15, oder zu einer der SEQ ID Nos. 16-18, oder zu einem antigenen Fragment davon.

3. Das Verfahren nach Anspruch 1, wobei das ein oder die mehreren Cytokine ausgewählt sind aus der Gruppe bestehend aus IL-1 beta, IL-6, IL-8, IL-10, IL-13, MCP-1, G-CSF, IFN-gamma, und TNF-alpha.

4. Das Verfahren nach Anspruch 1, wobei das Bestimmen des Stimulationsindexes das Messen der Aufnahme von tritiiertem Thymidin durch die Lymphozyten umfasst.

5. Das Verfahren nach Anspruch 1, wobei dass Bestimmen des Stimulationsindexes das in Kontakt bringen der Lymphozyten mit IFN-α umfasst.

6. Das Verfahren nach Anspruch 1, wobei das Messen eines oder mehrerer Cytokine die Durchführung eines Bioassays, eines Immunoassays, einer Durchflusscytometrie, oder eines Radioimmunoassays (RIA) umfasst.

7. Das Verfahren nach Anspruch 6, wobei der Immunoassay ein enzymgekoppelter Immunosorbens-Assay ist.

8. Das Verfahren nach Anspruch 1, wobei die Zusammensetzung ferner ein oder mehrere Polypeptide beinhaltet, abgeleitet von einer Art ausgewählt aus der Gruppe bestehend aus *Babesia bovis, Babesia divergens, Babesia microti, Bartonella bacilliformis, Bartonella henselae, Bartonella quintana, Bartonella rochalimae, Anaplasma phagocytophilum, Ehrlichia ewingii, Ehrlichia chaffeenis, Ehrlichia canis, Neorickettsia sennetsu, Mycoplasma fermentans, Mycoplasma hominis, Mycoplasma pneumoniae, Mycoplasma genitalium, Mycoplasma penetrans, Rickettsia rickettsii*, und *Rickettsia typhi*.

9. Ein Verfahren zum Bestimmen der Wahrscheinlichkeit Symptome, die mit Zeckenborreliose in Verbindung stehen, zu entwickeln in einem Testsubjekt, wobei das Verfahren umfasst:
a. Bestimmen eines Stimulationsindexes (SI) von Lymphozyten erhalten von einem Testsubjekt, wobei das Bestimmen das in Kontakt bringen einer Zusammensetzung mit den Lymphozyten umfasst, wobei die Zusammensetzung umfasst:
(i) ein im Wesentlichen gereinigtes Polypeptid umfassend eine Aminosäuresequenz mit mindestens 80% Sequenzidentität zu einer der SEQ ID Nos. 1-4, oder zu einem antigenen Fragment davon;
(ii) ein im Wesentlichen gereinigtes Polypeptid umfassend eine Aminosäuresequenz mit mindestens 80% Sequenzidentität zu SEQ ID No. 5, oder zu einem antigenen Fragment davon;
(iii) ein im Wesentlichen gereinigtes Polypeptid umfassend eine Aminosäuresequenz mit mindestens 80% Sequenzidentität zu einer der SEQ ID Nos. 6-7, oder zu einem antigenen Fragment davon;
(iv) ein im Wesentlichen gereinigtes Polypeptid umfassend eine Aminosäuresequenz mit mindestens 80% Sequenzidentität zu einer der SEQ ID Nos. 8-9, oder zu einem antigenen Fragment davon; und
b. Messen eines oder mehrerer Cytokine in einer Probe des Testsubjekts, wobei ein Anstieg in einem von SI oder Cytokin-Gehalt oder beiden relativ zu einer Kontrolle für eine erhöhte Wahrscheinlichkeit des Testsubjekts Symptome, die mit Zeckenborreliose in Verbindung stehen, zu entwickeln indikativ ist.

10. Ein Verfahren zur Überwachen der Behandlung von Zeckenborreliose in einem Testsubjekt, wobei das Verfahren umfasst:
a. Bestimmen einer Basislinie des Stimulationsindexes (SI) von Lymphozyten erhalten von einer Testsubjekt, wobei das Bestimmen das in Kontakt bringen einer Zusammensetzung mit den Lymphozyten aufweist, wobei die Zusammensetzung beinhaltet:
(i) ein im Wesentlichen gereinigtes Polypeptid umfassend eine Aminosäuresequenz mit mindestens 80% Sequenzidentität zu einer der SEQ ID Nos. 1-4, oder zu einem antigenen Fragment davon;
(ii) ein im Wesentlichen gereinigtes Polypeptid umfassend eine Aminosäuresequenz mit mindestens 80% Sequenzidentität zu SEQ ID No. 5, oder zu einem antigenen Fragment davon;
(iii) ein im Wesentlichen gereinigtes Polypeptid umfassend eine Aminosäuresequenz mit mindestens 80% Sequenzidentität zu einer der SEQ ID Nos. 6-7, oder zu einem antigenen Fragment davon;
(iv) ein im Wesentlichen gereinigtes Polypeptid umfassend eine Aminosäuresequenz mit mindestens 80% Sequenzidentität zu einer der SEQ ID Nos. 8-9, oder zu einem antigenen Fragment davon; und
b. Messen eines oder mehrerer Cytokine in einer Probe des Testsubjekts; und
c. Vergleichen eines späteren SI und Cytokin-Gehalts mit dem früheren SI und Cytokin-Gehalts nach der Behandlung des Testsubjekts gegen Zeckenborreliose, wobei eine Verringerung eines von SI und Cytokin-Gehalt oder beiden relativ zu dem früheren SI und Cytokin-Gehalt für eine positive Reaktion auf die Behandlung der Zeckenborreliose indikativ ist, und wobei keine Änderung oder ein Anstieg in einem von SI und Cytokin-Gehalt oder beiden relativ zu dem früheren SI und Cytokin-Gehalt für kein Ansprechen auf die Behandlung der Zeckenborreliose indikativ ist.

11. Eine Zusammensetzung zur Verwendung in dem Verfahren nach einem der Ansprüche 1 bis 10, die Zusammensetzung umfassend:
(a) ein im Wesentlichen gereinigtes Polypeptid umfassend eine Aminosäuresequenz mit mindestens 80% Sequenzidentität zu einer der SEQ ID Nos. 1-4, oder zu einem antigenen Fragment davon;
(b) ein im Wesentlichen gereinigtes Polypeptid umfassend eine Aminosäuresequenz mit mindestens 80% Sequenzidentität zu SEQ ID No. 5, oder zu einem antigenen Fragment davon;
(c) ein im Wesentlichen gereinigtes Polypeptid umfassend eine Aminosäuresequenz mit mindestens 80% Sequenzidentität zu einer der SEQ ID Nos. 6-7, oder zu einem antigenen Fragment davon;
(d) ein im Wesentlichen gereinigtes Polypeptid umfassend eine Aminosäuresequenz mit mindestens 80% Sequenzidentität zu einer der SEQ ID Nos. 8-9, oder zu einem antigenen Fragment davon.

12. Die Zusammensetzung nach Anspruch 11, umfassend:
(a) ein im Wesentlichen gereinigtes Polypeptid umfassend SEQ ID No. 1;
(b) ein im Wesentlichen gereinigtes Polypeptid umfassend SEQ ID No. 5;
(c) ein im Wesentlichen gereinigtes Polypeptid umfassend SEQ ID No. 7; und
(d) ein im Wesentlichen gereinigtes Polypeptid umfassend SEQ ID No. 8.

13. Die Zusammensetzung nach einem der Ansprüche 11 oder 12, wobei die Zusammensetzung ferner umfasst eines oder beide von:
(a) ein im Wesentlichen gereinigtes Polypeptid umfassend eine Aminosäuresequenz mit mindestens 80% Sequenzidentität zu einer der SEQ ID Nos. 10-12, oder zu einem antigenen Fragment davon; und
(b) ein im Wesentlichen gereinigtes Polypeptid umfassend eine Aminosäuresequenz mit mindestens 80% Sequenzidentität zu einer der SEQ ID Nos. 13-15, oder zu einer der SEQ ID Nos. 16-18, oder zu einem antigenen Fragment davon.

14. Die Zusammensetzung nach einem der Ansprüche 11 oder 12, wobei die Zusammensetzung ferner Interferon-alpha (IFN-α) umfasst.

15. Die Zusammensetzung nach einem der Ansprüche 11 oder 12, wobei die Zusammensetzung ferner ein oder mehrere Polypeptide umfasst, die abgeleitet sind von einer Art ausgewählt aus der Gruppe bestehend aus *Babesia bovis, Babesia divergens, Babesia microti, Bartonella bacilliformis, Bartonella henselae, Bartonella quintana, Bartonella rochadimae, Anaplasma phagocytophilum, Ehrlichia ewingii, Ehrlichia chaffeenis, Ehrlichia canis, Neorickettsia sennetsu, Mycoplasma fermentans, Mycoplasma hominis, Mycopdasma pneumoniae, Mycoplasma genitadium, Mycoplasma penetrans, Rickettsia rickettsii*, und *Rickettsia typhi*.

## Revendications

1. Procédé de diagnostic de la maladie de Lyme chez un sujet, le procédé comprenant :
a. la détermination d'un indice de stimulation (IS) de lymphocytes obtenus auprès du sujet,
dans lequel ladite détermination comprend la mise en contact d'une composition avec les lymphocytes, et dans lequel la composition comprend :
(i) un polypeptide sensiblement purifié comprenant une séquence d'acides aminés ayant au moins 80 % d'identité de séquence avec l'une quelconque des SEQ ID N° 1 à 4, ou avec un fragment antigénique de celles-ci ;
(ii) un polypeptide sensiblement purifié comprenant une séquence d'acides aminés ayant au moins 80 % d'identité de séquence avec SEQ ID N° 5, ou avec un fragment antigénique de celle-ci ;
(iii) un polypeptide sensiblement purifié comprenant une séquence d'acides aminés ayant au moins 80 % d'identité de séquence avec l'une quelconque des SEQ ID N° 6 à 7, ou avec un fragment antigénique de celles-ci ; et
(iv) un polypeptide sensiblement purifié comprenant une séquence d'acides aminés ayant au moins 80 % d'identité de séquence avec l'une quelconque des SEQ ID N° 8 à 9, ou avec un fragment antigénique de celles-ci ; et
b. la mesure d'une ou plusieurs cytokines dans un échantillon provenant du sujet, dans lequel une augmentation d'un ou des deux parmi IS et le taux de cytokine par rapport à un témoin est indicative de la maladie de Lyme chez le sujet.

2. Procédé selon la revendication 1, dans lequel la composition comprend en outre un quelconque ou les deux éléments suivants :
(a) un polypeptide sensiblement purifié comprenant une séquence d'acides aminés ayant au moins 80 % d'identité de séquence avec l'une quelconque des SEQ ID N° 10 à 12, ou avec un fragment antigénique de celles-ci ; et
(b) un polypeptide sensiblement purifié comprenant une séquence d'acides aminés ayant au moins 80 % d'identité de séquence avec l'une quelconque des SEQ ID N° 13 à 15, ou avec l'une quelconque des SEQ ID N° 16 à 18, ou avec un fragment antigénique de celles-ci.

3. Procédé selon la revendication 1, dans lequel les une ou plusieurs cytokines sont choisies dans le groupe consistant en IL-1 bêta, IL-6, IL-8, IL-10, IL-13, MCP-1, G-CSF, IFN-gamma, et TNF-alpha.

4. Procédé selon la revendication 1, dans lequel ladite détermination d'un indice de stimulation comprend la mesure de la capture de thymidine tritiée par les lymphocytes.

5. Procédé selon la revendication 1, dans lequel ladite détermination d'un indice de stimulation comprend la mise en contact des lymphocytes avec IFN-α.

6. Procédé selon la revendication 1, dans lequel la mesure d'une ou plusieurs cytokines comprend la réalisation d'un bio- essai, d'un immunoessai, d'une cytométrie en flux, ou d'un radioimmunoessai (RIA).

7. Procédé selon la revendication 6, dans lequel l'immunoessai est un essai d'immuno-absorption enzymatique.

8. Procédé selon la revendication 1, dans lequel la composition comprend en outre un ou plusieurs polypeptides dérivés d'une espèce choisie dans le groupe consistant en *Babesia bovis, Babesia divergens, Babesia microti, Bartonella bacilliformis, Bartonella henselae, Bartonella quintana, Bartonella rochalimae, Anaplasma phagocytophilum, Ehrlichia ewingii, Ehrlichia chaffeenis, Ehrlichia canis, Neorickettsia sennetsu, Mycop*/*asma fermentans, Mycop*/*asma hominis, Mycop*/*asma pneumoniae, Mycop*/*asma genitalium, Mycop*/*asma penetrans, Rickettsia rickettsii*, et *Rickettsia typhi*.

9. Procédé de détermination de la vraisemblance de développer des symptômes associés à la maladie de Lyme chez un sujet, le procédé comprenant :
a. la détermination d'un indice de stimulation (IS) de lymphocytes obtenus auprès du sujet, dans lequel ladite détermination comprend la mise en contact d'une composition avec les lymphocytes, dans lequel la composition comprend :
(i) un polypeptide sensiblement purifié comprenant une séquence d'acides aminés ayant au moins 80 % d'identité de séquence avec l'une quelconque des SEQ ID N° 1 à 4, ou avec un fragment antigénique de celle-ci ;
(ii) un polypeptide sensiblement purifié comprenant une séquence d'acides aminés ayant au moins 80 % d'identité de séquence avec SEQ ID N° 5, ou avec un fragment antigénique de celles-ci ;
(iii) un polypeptide sensiblement purifié comprenant une séquence d'acides aminés ayant au moins 80 % d'identité de séquence avec l'une quelconque des SEQ ID N° 6 à 7, ou avec un fragment antigénique de celles-ci ; et
(iv) un polypeptide sensiblement purifié comprenant une séquence d'acides aminés ayant au moins 80 % d'identité de séquence avec l'une quelconque des SEQ ID N° 8 à 9, ou avec un fragment antigénique de celles-ci ; et
b. la mesure d'une ou plusieurs cytokines dans un échantillon provenant du sujet, dans lequel une augmentation d'un ou des deux parmi IS et le taux de cytokine par rapport à un témoin est indicative d'une vraisemblance accrue de développer des symptômes associés à la maladie de Lyme chez le sujet.

10. Procédé de contrôle du traitement de la maladie de Lyme chez un sujet, le procédé comprenant
a. la détermination d'un indice de stimulation (IS) de ligne de base de lymphocytes obtenus auprès du sujet, dans lequel ladite détermination comprend la mise en contact d'une composition avec les lymphocytes, dans lequel la composition comprend :
(i) un polypeptide sensiblement purifié comprenant une séquence d'acides aminés ayant au moins 80 % d'identité de séquence avec l'une quelconque des SEQ ID N° 1 à 4, ou avec un fragment antigénique de celles-ci ;
(ii) un polypeptide sensiblement purifié comprenant une séquence d'acides aminés ayant au moins 80 % d'identité de séquence avec SEQ ID N° 5, ou avec un fragment antigénique de celle-ci ;
(iii) un polypeptide sensiblement purifié comprenant une séquence d'acides aminés ayant au moins 80 % d'identité de séquence avec l'une quelconque des SEQ ID N° 6 à 7, ou avec un fragment antigénique de celles-ci ; et
(iv) un polypeptide sensiblement purifié comprenant une séquence d'acides aminés ayant au moins 80 % d'identité de séquence avec l'une quelconque des SEQ ID N° 8 à 9, ou avec un fragment antigénique de celles-ci ;
b. la mesure d'une ou plusieurs cytokines dans un échantillon provenant du sujet ; et
c. la comparaison d'un IS et d'un taux de cytokine ultérieurs à un IS et un taux de cytokine antérieurs après traitement du sujet pour la maladie de Lyme, dans lequel une diminution d'un de l'IS ou du taux de cytokine ou des deux par rapport à l'IS et au taux de cytokine antérieurs est indicative d'une réponse positive au traitement de la maladie de Lyme, et dans lequel pas de changement ou une augmentation dans l'un de l'IS et du taux de cytokine ou des deux par rapport à l'IS et au taux de cytokine antérieurs est indicatif (indicative) d'une réponse nulle au traitement de la maladie de Lyme.

11. Composition à utiliser dans un procédé de l'une quelconque des revendications 1 à 10, la composition comprenant :
(a) un polypeptide sensiblement purifié comprenant une séquence d'acides aminés ayant au moins 80 % d'identité de séquence avec l'une quelconque des SEQ ID N° 1 à 4, ou avec un fragment antigénique de celles-ci ;
(b) un polypeptide sensiblement purifié comprenant une séquence d'acides aminés ayant au moins 80 % d'identité de séquence avec SEQ ID N° 5, ou avec un fragment antigénique de celle-ci ;
(c) un polypeptide sensiblement purifié comprenant une séquence d'acides aminés ayant au moins 80 % d'identité de séquence avec l'une quelconque des SEQ ID N° 6 à 7, ou avec un fragment antigénique de celles-ci ; et
(d) un polypeptide sensiblement purifié comprenant une séquence d'acides aminés ayant au moins 80 % d'identité de séquence avec l'une quelconque des SEQ ID N° 8 à 9, ou avec un fragment antigénique de celles-ci.

12. Composition selon la revendication 11, comprenant :
(a) un polypeptide sensiblement purifié comprenant SEQ ID N° 1 ;
(b) un polypeptide sensiblement purifié comprenant SEQ ID N° 5 ;
(c) un polypeptide sensiblement purifié comprenant SEQ ID N° 7 ; et
(d) un polypeptide sensiblement purifié comprenant SEQ ID N° 8.

13. Composition selon l'une quelconque des revendications 11 ou 12, dans laquelle la composition comprend en outre l'un ou les deux parmi :
(a) un polypeptide sensiblement purifié comprenant une séquence d'acides aminés ayant au moins 80 % d'identité de séquence avec l'une quelconque des SEQ ID N° 10 à 12, ou avec un fragment antigénique de celles-ci ; et
(b) un polypeptide sensiblement purifié comprenant une séquence d'acides aminés ayant au moins 80 % d'identité de séquence avec l'une quelconque des SEQ ID N° 13 à 15, ou avec l'une quelconque des SEQ ID N° 16 à 18, ou avec un fragment antigénique de celles-ci.

14. Composition selon l'une quelconque des revendications 11 ou 12, dans laquelle la composition comprend en outre l'interféron-alpha (IFN-α).

15. Composition selon l'une quelconque des revendications 11 ou 12, dans laquelle la composition comprend en outre un ou plusieurs polypeptides dérivés d'une espèce choisie dans le groupe consistant en *Babesia bovis, Babesia divergens, Babesia microti, Bartonella bacilliformis, Bartonella henselae, Bartonella quintana, Bartonella rochalimae, Anaplasma phagocytophilum, Ehrlichia ewingii, Ehrlichia chaffeenis, Ehrlichia canis, Neorickettsia sennetsu, Mycop*/*asma fermentans, Mycop*/*asma hominis, Mycop*/*asma pneumoniae, Mycop*/*asma genitalium, Mycop*/*asma penetrans, Rickettsia rickettsii,* et *Rickettsia typhi*.
